# EUROPEAN PATENT SPECIFICATION

(11) **EP 1 962 948 B1**
(45) Date of publication and mention of the grant of the patent: **22.10.2014**
(21) Application number: 06815485.5
(22) Date of filing: 26.09.2006
(51) Int. Cl.: H01R 13/18

(54) **LEAD INTERCONNECTIONS AND REINFORCEMENT**
DRAHTVERBINDUNGEN UND VERSTÄRKUNG
INTERCONNEXIONS ET RENFORCEMENT DE FILS

(30) Priority: 19.12.2005 US 305925
(43) Date of publication of application: 03.09.2008
(73) Proprietor: Cardiac Pacemakers, Inc., St. Paul MN 55112-5798 (US)
(72) Inventor: ZAREMBO, Paul E., Vadnais Heights, MN 55127 (US); KRISHNAN, Mohan, Shoreview, MN 55126 (US); DURAND, David, Osceola, WI 54020 (US)
(74) Representative: Golkowsky, Stefan
(86) International application number: PCT/US2006/037527
(87) International publication number: WO 2007/078360

(56) References cited:
- EP-A1- 0 778 048
- WO-A2-00/27469
- FR-A- 2 508 716
- US-A- 4 214 804
- US-A- 5 014 720
- US-A- 5 231 996
- US-A- 5 251 643
- US-A- 5 347 708
- US-A- 5 487 757
- US-A1- 2002 147 484
- US-A1- 2004 014 355
- US-A1- 2004 064 174
- US-A1- 2005 228 469
- US-B1- 6 366 820

## Description

### TECHNICAL FIELD

This patent document pertains generally to leads for linking medical devices with selected body tissue to be sensed or stimulated by such devices. More particularly, but not by way of limitation, this patent document pertains to interconnections of lead conductors and electrodes and reinforcement therefor.

### BACKGROUND

Implantable leads represent the electrical link between an implantable medical device (often referred to simply as an "IMD") and a subject's cardiac or other tissue, which is to be sensed or stimulated. An implantable lead may include a single or multiple conductors that are connected to an electrode or an electrode assembly at a lead intermediate portion or a lead distal end portion. A connector assembly is included at a lead proximal end portion to form an electrical connection, via the conductor(s), between the electrode or electrode assembly and the IMD.

Over the years, a large number of different mechanisms and methods for interconnecting conductors and electrodes have been proposed. It is desirable that such connections between the conductors and the electrodes provide a highly reliable electrical connection, with good mechanical properties including high tensile strength. It is also desirable that such connections allow for the lead assembly itself to retain a high degree of tensile strength through the areas of the electrodes. This is because cardiac and other leads undergo considerable stresses due to repetitive flexing caused by, for example, the motion of a beating heart and forces applied to the lead during an implantation, repositioning, or lead extraction procedure.

Typically, conductors in commercially marketed pacing and defibrillation leads have taken the form of single or multi-filar wire coils. Recently, there has been a high level of interest in designing leads having lead bodies with a reduced size (i.e., lead body diameter) or additional electrodes. One way to reduce to lead body size is to employ, at least in part, stranded wire conductors such as cables, in lieu of coiled wire conductors. However, such stranded wire conductors present new challenges not faced by the use of coiled wire conductors. As one example, it has been a great challenge to electrically and reliably connect a small multi-strand conductor cable - often times having a cable outer diameter on the order of thousandths of an inch - to a ring electrode or a multi-filar shock coil electrode. Being of such small size, the connection is a very difficult one to make and fragile, if made incorrectly.

With respect to single or multi-filar wire coiled conductors, when such conductors are used to electrically connect a distal tip electrode to the IMD, portions of the distal electrode typically are polymer bonded (e.g., via an adhesive) to provide additional axial strength to the electrode. However, even with such additional polymer-provided strength, the distal electrode/lead body connection may still fall short of the axial strength necessary to resist permanent deformation or in order to pass industry standards (e.g., CEN/CENELEC 45502-2-1, § 23.3).

Document WO 00/27469 discloses a lead according to the preamble of claim 1.

### SUMMARY

A lead comprises a lead body extending from a lead proximal end portion to a lead distal end portion. The lead body includes one or more longitudinally extending lumens. A conductor is received in, and extends along, a lumen. In varying examples, the lead further comprises a tubular electrode co-axial with, and overlying portions of, the lead body. In one example, a lumen wall is sized and shaped to urge an electrically conductive interposer coupled with the conductor toward an inner surface of the electrode.

In a further example, an axial support member provides reinforcement support between a distal end electrode and the lead body. Methods associated with the foregoing are also discussed.

In Example 1, a lead comprises a lead body extending from a lead proximal end portion to a lead distal end portion and having a lead intermediate portion therebetween, the lead body including one or more longitudinally extending lumens; a conductor received in, and extending along, a first lumen; an electrically conductive interposer coupled with the conductor, the interposer received, at least in part, in the first lumen; a tubular electrode co-axial with, and overlying portions of, the lead body; and a portion of a first lumen wall compressively urging the interposer, at least in part, toward an inner surface of the tubular electrode.

In Example 2, the lead of Example 1 optionally includes an interposer exposing aperture in the first lumen wall disposed opposite the first lumen wall urging portion.

In Example 3, the lead of Examples 1-2 is optionally configured such that the interposer is coupled with an inner surface of the tubular electrode.

In Example 4, the lead of Examples 1-3 is optionally configured such that an outer surface of the interposer mates with a portion of the inner surface of the tubular electrode.

In Example 5, the lead of Examples 1-4 is optionally configured such that the lead body comprises an elastic polymer.

In Example 6, the lead of Examples 1-5 is optionally configured such that the conductor comprises a cable conductor.

In Example 7, a method comprises forming a lead body extending from a lead proximal end portion to a lead distal end portion and having a lead intermediate portion therebetween; forming one or more longitudinally extending lumens within the lead body, including forming a first lumen having: a lumen wall portion sized and shaped to urge an electrically conductive interposer toward an inner surface of a tubular electrode co-axial with, and overlying portions of, the lead body, and a lumen wall portion comprising an interposer exposing aperture sized and shaped to allow electrical connection between the interposer and the tubular electrode; drawing a conductor into and through the first lumen; inserting the interposer, at least in part, into the first lumen; and coupling the interposer with the conductor.

In Example 8, the method of Example 7 optionally includes compression fitting the interposer with the inner surface of the tubular electrode.

In Example 9, the method of Example 8 is optionally configured such that the compression fitting of the interposer with the inner surface of the tubular electrode includes disposing a removable expanding mandrel in a second lumen positioned adjacent the first lumen; and urging the interposer toward the inner surface of the electrode via movement of the mandrel.

In Example 10, the method of Examples 7-9 optionally includes coupling an outer surface of the interposer with the inner surface of the tubular electrode.

In Example 15, the lead of Examples 11-14 is optionally configured such that the conductor comprises a coil conductor.

In Example 16, a method comprises forming a lead body extending from a lead proximal end portion to a lead distal end portion and having a lead intermediate portion therebetween, including forming a longitudinally extending lumen within the lead body; disposing a ring member within the lumen, including inserting a portion of the ring member through an aperture in a lumen wall; drawing a conductor into and through the lumen, including disposing a distal end portion of the conductor adjacent to the ring member; coupling the conductor with the ring member; and overlying portions of the lead body with a tubular electrode, the tubular electrode co-axial with the lead body and electrically coupled with the conductor.

In Example 17, the method of Example 16 is optionally configured such that the coupling the conductor with the ring member includes disposing a securing member around the distal end portion of the conductor and the ring member; and deforming the securing member over the conductor.

In Example 18, the method of Examples 16-17 optionally includes forming one or more grooves on the ring member; and wherein coupling the conductor with the ring member includes using the one or more grooves.

In Example 19, the method of Examples 16-18 is optionally configured such that the coupling the conductor with the ring member includes using a conductive adhesive.

In Example 20, the method of Examples 16-19 is optionally configured such that the overlying portions of the lead body with the tubular electrode includes forming a substantially isodiametric surface at an interface of the tubular electrode and an outer surface of the lead body.

In Example 21, a lead comprises a lead body extending from a lead proximal end portion to a lead distal end portion and having a lead intermediate portion therebetween, the lead body including at least one of a lumen or a slit; a first conductor and a second conductor; and an electrically conductive connector having a first end portion coupled with the first conductor and having a second end portion coupled with the second conductor, the connector received in the lumen or the slit.

In Example 22, the lead of Example 21 optionally includes a securing member disposed around, and deformed over, a portion of one or both of the first conductor or the second conductor.

In Example 23, the lead of Examples 21-22 is optionally configured such that one or both of the first end portion or the second end portion include one or more grooves to which the first or second conductor are coupled.

In Example 24, the lead of Example 23 is optionally configured such that the one or more grooves are disposed on an inner surface of the connector.

In Example 25, the lead of Example 23 is optionally configured such that the one or more grooves are disposed on an outer surface of the connector.

In Example 11 an axial support member received in a second lumen, the axial support member extending from an axial support member proximal end portion to an axial support member distal end portion; the axial support member proximal end portion being coupled with a first retaining member and the axial support member distal end portion being coupled with a second retaining member; and the first retaining member being engaged with the lead body and the second retaining member being engaged with the distal electrode.

In Example 12, the lead of Example 11 is optionally configured such that one or both of the first retaining member or the second retaining member includes a couplable tube.

In Example 13, the lead of Examples 11-12 is optionally configured such that an outer diameter of one or both of the first or the second retaining member is greater than a diameter of the second lumen.

The leads and methods described herein provide numerous advantages over conventional lead designs including secure electrical and mechanical connection between a conductor, such as a multi-strand conductor cable, and an electrode (e.g., a ring electrode or a multi-filar shock coil electrode). In addition, the leads and methods provide axial reinforcement between a distally disposed electrode and a lead body. Furthermore, the leads and methods allow for the creation of a relatively small-sized lead (e.g., sub-5 French), which advantageously may provide for easier and deeper lead delivery and lower sensing/stimulating thresholds. In one such example, the present leads and methods provide a small-sized lead with multiple conductors and electrodes. Multiple conductors and electrodes allow for electrode switching to prevent extra bodily tissue stimulation and optimize a variety of other sensing/stimulating related parameters (e.g., stimulation threshold parameter, stimulation impedance parameter, stimulation selection parameter, sense voltage parameter, sense noise parameter, tissue electrode location parameter, heart chamber configuration parameter, blood flow parameter, posture parameter, blood volume parameter, acceleration or motion parameter, spatial distance parameter, timing parameter, impedance parameter, blood oxygen level parameter, stimulation energy parameter, among others).

Several other advantages are also made possible by the present leads and methods. In some examples, the leads and methods reduce or eliminate the reliance on adhesives to couple a conductor to an electrode. Advantageously, by reducing or eliminating reliance on adhesives, manufacturing efficiency can be increased (e.g., may not need to wait for adhesives to cure), and conductor/electrode joint failure caused by adhesive bond strength decreasing over time (e.g., due to reactions with bodily fluids or improper adhesive preparation) can be reduced or eliminated. These and other examples, aspects, advantages, and features of the leads and methods described herein will be set forth in part in the detailed description, which follows, and in part will become apparent to those skilled in the art by reference to the following description of the present leads and methods, and drawings or by practice of the same.

### BRIEF DESCRIPTION OF THE DRAWINGS

In the drawings, which are not necessarily drawn to scale, like numerals describe similar components throughout the several views. The drawings illustrate generally, by way of example, but not by way of limitation, various embodiments discussed in this patent document.
- FIG. **1**: is a schematic view illustrating an implantable lead system and an environment in which the lead system may be used, as constructed in accordance with at least one embodiment.
- FIG. **2A**: is a schematic view illustrating an implantable lead system for delivering or receiving signals to and from a heart, as positioned and constructed in accordance with at least one embodiment.
- FIG. **2B**: is a schematic view illustrating an implantable lead system for delivering or receiving signals to and from a heart, as positioned and constructed in accordance with at least one embodiment.
- FIG. **3**: is a plan view of an implantable lead, as constructed in accordance with at least one embodiment.
- FIG. **4**: is a longitudinal cross-sectional view of an implantable lead, as constructed in accordance with at least one embodiment.
- FIG. **5**: is a longitudinal cross-sectional view of a portion of an implantable lead, as constructed in accordance with at least one embodiments.
- FIG. **6A**: is a transverse cross-sectional view of an implantable lead taken along line **6A-6A** of FIG. **5****,** as constructed in accordance with at least one embodiment.
- FIG. **6B**: is a longitudinal cross-sectional view of a portion of an implantable lead, as constructed in accordance with at least one embodiment.
- FIG. **6C**: is a transverse cross-sectional view of an implantable lead taken along line **6A-6A** of FIG. **5****,** as constructed in accordance with at least one embodiment.
- FIG. **6D**: is a transverse cross-sectional view of an implantable lead including an urging member taken along line **6A-6A** of FIG. **5****,** as constructed in accordance with at least one embodiment.
- FIG. **7A**: is a longitudinal cross-sectional view of a portion of an implantable lead, as constructed in accordance with at least one embodiment.
- FIG. **7B**: is a transverse cross-section view of an implantable lead taken along line **10B-10B** of FIG. **10A**, as constructed in accordance with at least one embodiment.
- FIG. **8**: illustrates a method of manufacturing an implantable lead, as constructed in accordance with at least one embodiment.

### DETAILED DESCRIPTION

The following detailed description includes references to the accompanying drawings, which form a part of the detailed description. The drawings show, by way of illustration, specific embodiments in which the present leads and methods may be practiced. These embodiments, which are also referred to herein as "examples," are described in enough detail to enable those skilled in the art to practice the present leads and methods. The embodiments may be combined or varied, other embodiments may be utilized or structural or logical changes may be made without departing from the scope of the present leads and methods. It is also to be understood that the various embodiments of the present leads and methods, although different, are not necessarily mutually exclusive. For example, a particular feature, structure or characteristic described in one embodiment may be included within other embodiments. The following detailed description is, therefore, not to be taken in a limiting sense, and the scope of the present leads and methods are defined by the appended claims and their equivalents.

In this document the terms "a" or "an" are used to include one or more than one; the term "or" is used to refer to a nonexclusive or, unless otherwise indicated; and the term "subject" is used synonymously with the term "patient."

Leads and methods discussed herein advantageously provide for secure electrical and mechanical connections between a conductor and an electrode or a conductor and another conductor, while further providing a small lead body diameter. In one example, a lead includes a cable conductor/electrode connection design using the compressive or elastic nature of a polymer lead body in conjunction with an appropriately larger sized electrically conductive interposer (e.g., metallic tube).

In a further example, a lead includes an axial support member to reinforce a connection between a distally disposed electrode and a portion of the lead body.

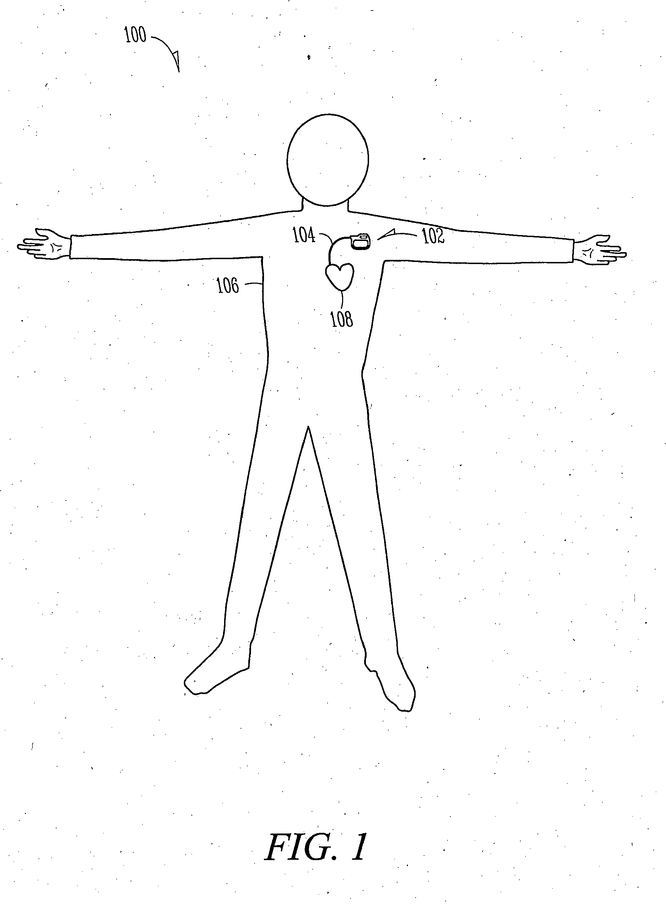

Turning now to the drawings, and initially to FIG. **1****,** which illustrates a lead system **100** and an environment **106** (e.g., subcutaneous pocket made in the wall of a subject's chest, abdomen, or elsewhere) in which the lead system **100** may be used. In varying examples, the lead system **100** may be used for delivering or receiving electrical pulses or signals to stimulate or sense a heart **108** of a subject. As shown in FIG. **1****,** the lead system **100** includes an implantable medical device (referred to as an "IMD") **102** and an implantable lead **104.** The IMD **102** includes a source of power as well as an electronic circuitry portion. In this example, the IMD **102** is a battery-powered device that senses intrinsic signals of the heart **108** and generates a series of timed electrical discharges. The IMD **102** generically represents, but is not limited to, cardiac rhythm management devices (referred to as "CRM devices") such as pacers, cardioverters, biventricular/cardiac resynchronization paces, defibrillators, or sensing instruments.

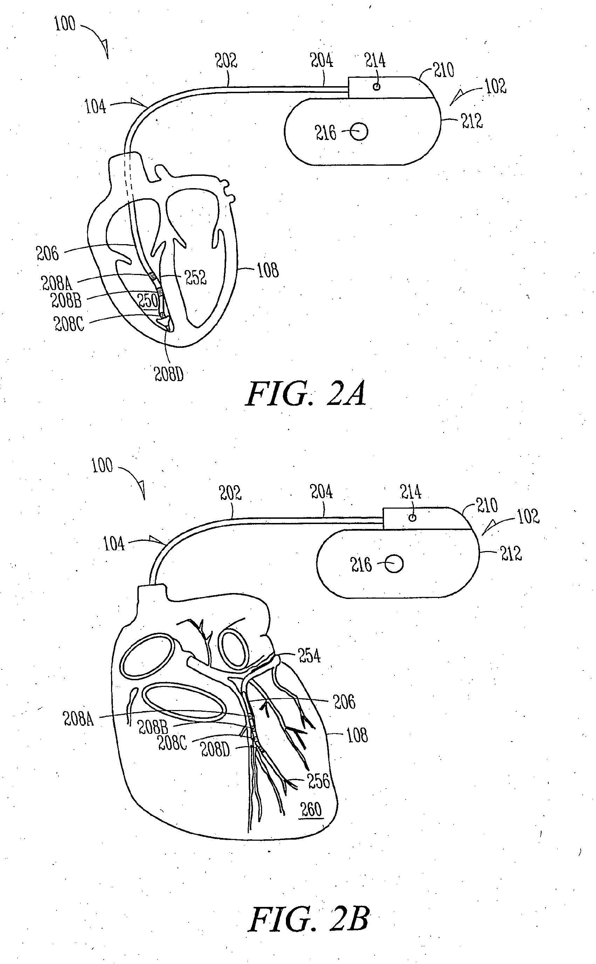

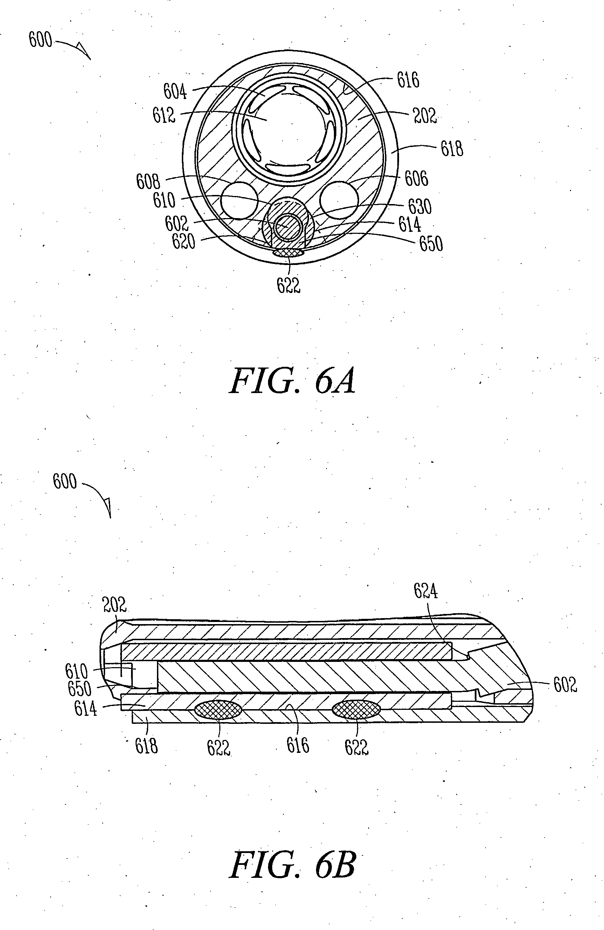

FIGS. **2A-2B** are schematic views of a lead system **100** including an IMD **102** and at least one lead **104.** The lead **104** includes a lead body **202** which extends from a lead proximal end portion **204,** where it is coupled with the IMD **102,** to a lead distal end portion **206,** where it is coupled with a portion of a subject's heart **108,** for example, via entanglement, lodging, or helical fixation when implanted. The lead distal end portion **206** includes at least one electrode **208***A*, **208***B*, **208***C*, **208***D* that electrically couples the lead **104** with the heart **108.** At least one conductor cable **602** or coil **604** (both shown in FIG. **6A**), electrically couple the electrodes **208***A***, 208***B*, **208***C*, **208***D* with the lead proximal end portion **204** and, thus the IMD **102.** The conductors **602, 604** (FIG. **6A**) carry electrical current, pulses or shocks between the IMD **102** and the electrodes **208***A*, **208***B***, 208***C*, **208***D*. The lead **104** may be installed using both over-the-wire techniques or non-over-the wire techniques (e.g., stylet driven techniques or catheter delivered techniques).

In the examples shown in FIGS. **2A-2B****,** the lead **104** is a multi-electrode lead that includes a proximal electrode **208***A*, two intermediate electrodes **208***B***, 208***C*, and a distal electrode **208***D*. Each of the electrodes **208***A***, 208***B***, 208***C*, **208***D* may be ring electrodes or multi-filar shock coil electrodes and are independently electrically connected to a separate corresponding electrically conductive terminal within a header **210** of the IMD **102.** The header **210** is affixed to a hermetically sealed housing **212,** which may be formed from a conductive metal such as titanium, and which carries, at least portions of, the electronic circuitry of the IMD **102.** In this example, the header **210** includes a header electrode **214** and the housing **212** includes a housing electrode **216,** both of which may be used in one or more electrode configurations for sensing or stimulating heart **108.**

As shown in FIG. **2A****,** the lead distal end portion **206** of the lead **104** is disposed in a right ventricle **250** of the heart **108.** FIG. **2A** further illustrates that the lead **104** may include at least one preformed biased portion to urge one or more electrodes thereon against a septal wall **252** of the heart **108** for pacing or sensing of the same. Referring now to FIG. **2B**, the lead distal end portion **206 is** disposed in a coronary vein **256** after being guided through a coronary sinus ostium and a coronary sinus **254.** Placing the lead **104** in a coronary branch-vein (e.g., **256**) on a left ventricle **260** has been found to be a suitable means for delivering stimulation therapy to a subject suffering from congestive heart failure without having to position the lead **104** within the left ventricle **260**. Other lead **104** placements besides those illustrated in FIGS. **2A-2B** are also possible without departing from the scope of the present leads and methods.

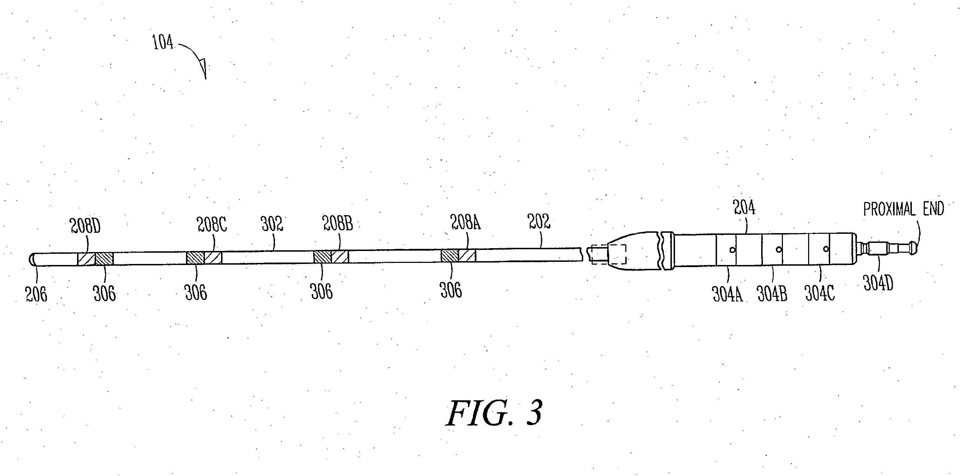

FIG. **3** illustrates a plan view of an implantable lead **104.** As shown, the lead **104** includes a lead body **202** extending from a lead proximal end portion **204** to a lead distal end portion **206** and having an intermediate portion **302** therebetween. In one example, the lead body **202** comprises biocompatible tubing such as medical grade polyurethane. In another example, the lead body **202** comprises medical grade silicone rubber or other thermoplastic or polymer known in the art to be suitable for use in leads. As discussed above in association with FIG. **1****,** a lead system **100** includes, among other things, the lead **104** for electrically coupling an IMD **102** (FIG. **1**) to bodily tissue, such as a heart **108** (FIG. **1**), which is to be excited (i.e., stimulated) or sensed by one or more electrodes **208***A***, 208***B***, 208***C*, **208***D*. It should also be understood that the lead **104** may also include means for sensing other physiological parameters, such as pressure, oxygen saturation, temperature, or the like. The lead **104** may include electrodes **208***A***, 208***B***, 208***C***, 208***D* only, other physiologic sensors, drug collars **306**, or a combination thereof.

In the example shown in FIG. **3****,** the lead proximal end portion **204** includes four terminal connections **304***A****,* 304***B****,* 304***C*, **304***D* disposed therealong. Similarly, the lead intermediate portion **302** or the lead distal end portion **206** includes four electrodes **208***A****,* 208***B*, **208***C*, **208***D* disposed therealong. The electrodes **208***A,* **208***B***, 208***C*, **208***D* are each adapted to sense or stimulate the heart **108** (FIG. **1**) and are electrically coupled to the terminal connections **304***A****,* 304***B*, **304***C****,* 304***D* via at least four conductor cables **602** or coils **604** (both shown in FIG. **6A**) contained within the lead body **202**, such as in one or more internal longitudinally extending lumens **606, 608, 610, 612** (FIG. **6A**). The lead proximal end portion **204** and the terminal connections **304***A*, **304***B****,* 304***C*, **304***D* disposed therealong are sized and shaped to couple to a multi-pole connector cavity, which may be incorporated into a header **210** (FIGS. **2A-2B**) of the IMD **102** (FIGS. **2A-2B**). It is through the coupling between the lead proximal end portion **204** and the multi-polar connector cavity that electrodes **208***A*, **208***B*, **208***C,* **208***D* are electrically coupled to electronic circuitry of the IMD **102.**

Advantageously, the present leads and methods provide for secure electrical and mechanical connection between the conductor cables **602** or coils **604** (both shown in FIG. **6A**) and the electrodes **208***A*, **208***B*, **208***C*, **208***D* while maintaining a relatively small-sized lead body **202,** such as a lead body sub-5 French in diameter. In one example, as discussed in greater detail in association with FIGS. **6A** and **6B**, the lead **104** includes a conductor/electrode connection design using the compressive nature of the lead body **202** in conjunction with an appropriately sized electrically conductive interposer (e.g., electrically conductive tube) **614** (FIG. **6A**) or similar element.

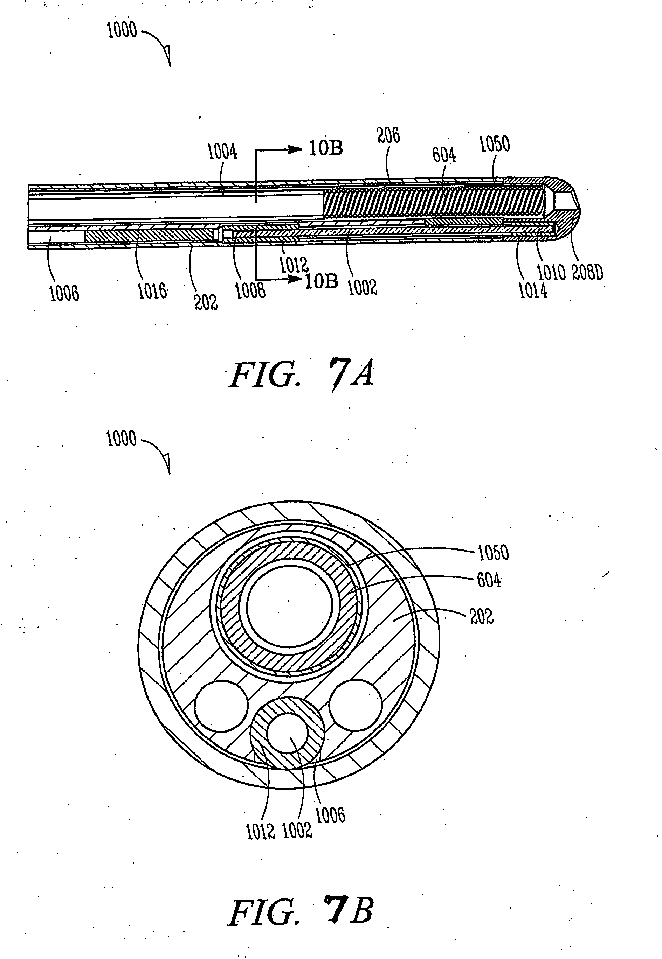

In a further example, as discussed in greater detail in association with FIGS. **7A** and **7B**, the lead **104** includes an axial support member **1002** to reinforce a connection between a distal electrode **208D** and a portion of the lead body **202.**

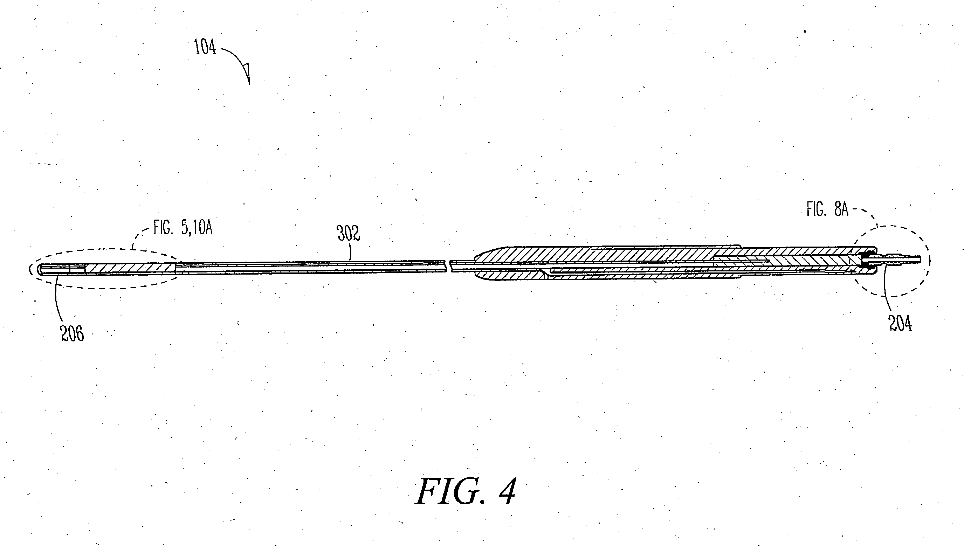

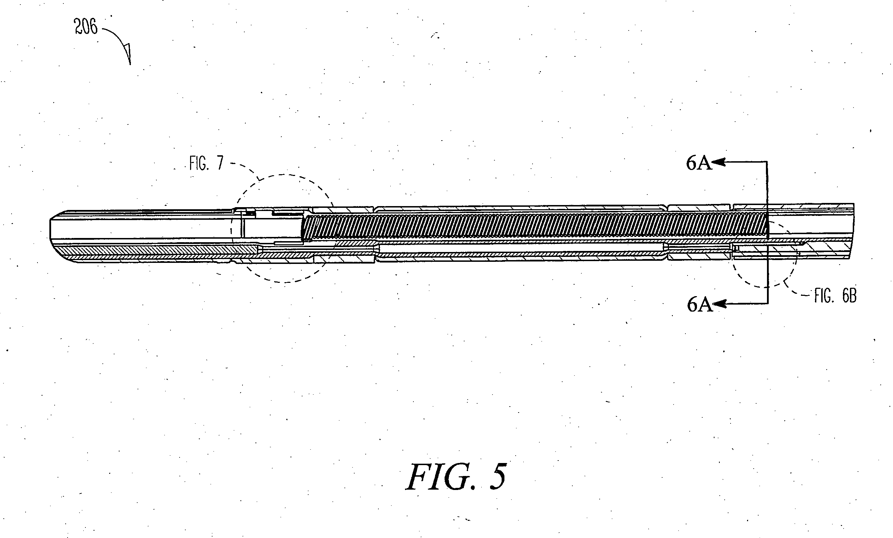

FIGS. 4, 5, and **7A** illustrate cross-sectional views of an implantable lead 104 or portions thereof. Specifically, FIG. 4 illustrates a longitudinal cross-sectional view of an implantable lead **104** extending from a lead proximal end portion **204** to a lead distal end portion **206** and having a lead intermediate portion **302** therebetween. FIG. **5** is a longitudinal cross-sectional view illustrating a lead distal end portion **206** or a lead intermediate portion **302** of an implantable lead **104** (FIG. **4**) in greater detail. FIG. **7A** is a longitudinal cross-sectional view illustrating a lead distal end portion **206** or a lead intermediate portion **302** of an implantable lead **104** (FIG. **4**) in greater detail.

FIG. **6A** illustrates a transverse cross-sectional view taken along line **6A-6A** of FIG. 5 of an implantable lead **104** (FIG. **4**). FIG. **6A** shows, among other things, a conductor/electrode connection design **600** using the compressive nature of a lead body **202** in conjunction with an appropriately sized (i.e., slightly larger than a size of a receiving lumen **610**) electrically conductive interposer (e.g., electrically conductive tube) **614,** and further in conjunction with an interposer exposing hole or slit (collectively termed "aperture") **650** in a wall of the lumen **610.** In this example, the lead body **202** includes four internal longitudinally extending lumens **606, 608, 610, 612** that allow one or more conductor cables **602** or coils **604** to be received in, and extend along, the lead **104** (FIG. **4**). In one example, wall portions of at least one lumen (e.g., **610**) are sized and shaped to urge an electrically conductive interposer **614** toward an inner surface **616** of a tubular electrode **618** co-axial with, and overlying portions of, the lead body **202** through a hole or slit **650.** When the slightly larger than lumen **610** electrically conductive interposer **614** is inserted into the lumen **610,** the wall portions of the lumen deform into an urging shape, such as that depicted by phantom line **630.**

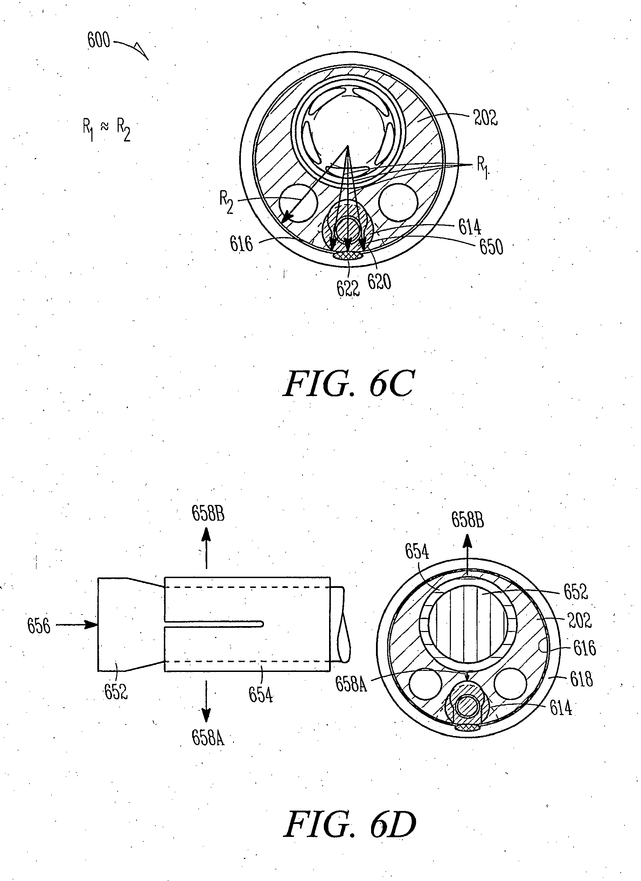

In this example, the electrically conductive interposer **614** is a metal tube that is coupled with the conductor **602** and inserted into the at least one lumen (e.g., **610**) having an urging size and shape. The coupling between the electrically conductive interposer **614** and the conductor **602** may be performed by, among other techniques, crimping, swaging, welding, or brazing. In one such example, as shown in FIG. **6C****,** the electrically conductive interposer **614** is crimped to the conductor **602** (FIG. **6B**) such that an outer surface **620** of tube **614** mates, in part, with an inner surface **616** of the tubular electrode **618** (FIG. **6B**) (i.e., R₁, representing a radius from a lead body **202** center to the outer surface **620,** is approximately equal to R₂, representing a radius from the lead body **202** center to the inner surface **616**). This ensures intimate contact and an adequate fitting for welding **622** the tubular electrode **618** to the electrically conductive interposer **614**. After being inserted in the lumen **610** and urged toward the inner surface **616** of tubular electrode **618 by** wall portions of the lumen **610** through the hole or slit **650,** the electrically conductive interposer **614** may be coupled to the tubular electrode **618,** such as by spot or laser welding **622** (see also FIG. **6B**), brazing, or using a conductive adhesive.

FIG. **6B** is a longitudinal cross-sectional view illustrating a lead distal end portion **206** or a lead intermediate portion **302** of an implantable lead **104.** Specifically, FIG. **6B** illustrates one or more spot or laser weldings **622** that may be made between an electrically conductive interposer **614** and a tubular electrode **618,** in accordance with at least one embodiment of a conductor/electrode connection design **600.** As shown in FIG. **6B****,** wall portions of a lumen **610** in the lead body **202** are sized and shaped to urge the electrically conductive interposer **614** toward an inner surface **616** of the tubular electrode **618** through the hole or slit **650.** As also shown, the electrically conductive interposer **614** is coupled with a conductor **602,** such as by crimping, swaging, welding, or brazing. Prior to the crimping, swaging, welding, or brazing, most of the insulating coating **624** on a distal end of the conductor **602** may need to be removed to allow for electrical communication between the electrically conductive interposer **614** and the conductor **602**. Although not shown in FIG. **6B****,** the weld **622** may be a continuous weld between the interposer **614** and the electrode **618.**

FIG. **6D** illustrates a removable expanding mandrel **652** surrounded by a split cylinder **654,** both of which may be inserted into a coil receiving lumen **612** (FIG. **6A**) of a lead body **202** to urge an electrically conductive interposer **614** toward an inner surface **616** of a tubular electrode **618.** In one example, movement of the mandrel **652** in a direction **656** causes the split cylinder **654** to expand in the directions **658***A***, 658***B***,** which in turn urges lead body **202** against interposer **614** in a direction toward electrode **618.**

over a slightly larger diameter ring member **706.** In another example, a securing member **702** is disposed around the distal portion of the conductor **604** and the ring member **706** is deformed, such as by crimping, rotary swaging, welding, or brazing. Rotary swaging is a metal forming process for the diametrical reduction of annular securing members, such as bars, tubes, wires, etc. In this example, the securing member **702** is deformed over the distal end portion of the conductor **604** thereby coupling the conductor **604** to the ring member **706.** As shown in FIG. **7A****,** a tubular electrode **618** co-axial with, and overlying portions of, the lead body **202** is electrically coupled with the conductor **604** via the conductive securing member **702** and/or the conductive ring member **706.** In one example, a removable preformed mandrel **752** is used to urge the ring member **706** toward the electrode **618** prior to coupling between the same. As shown, the mandrel **752** includes a preformed bend **760** to urge the ring member **706** toward the electrode **618.**

In the example of FIG. **7B**, an adhesive **708** and one or more grooves or threads **710** are used in conjunction with a securing member **702** to couple a conductor **604** to a ring member **706.** As discussed above, after the securing member **702** is positioned over a distal end portion of the conductor **604** and the ring member **706,** the securing member **702** may then deformed to snugly secure the conductor **604** to the ring member **706.** The deformation decreases the internal diameter of the securing member **702** thereby reconfiguring a shape of the securing member **702** and pinching the adhesive **708,** which may be inserted for additional coupling strength, over the conductor **604** and into the one or more grooves or threads **710** of the ring member **706.** The grooves or threads **710** may be used to provide additional axial strength at the conductor/electrode connection design **700.** The adhesive **708** may be a suitable medical grade adhesive, such as silicone based adhesive, a two-part adhesive, a conductive adhesive, or another similar adhesive. The adhesive **708** forms a secure mechanical bond between the securing member **702,** the conductor **604,** and the ring member **710.** As shown in FIG. **7B**, a tubular electrode **618** co-axial with, and overlying portions of, the lead body **202** is electrically coupled with the conductor **604** via the conductive securing member **702** and/or the conductive ring member **706.**

FIG. **7A** illustrates a longitudinal cross-sectional view of a lead distal end portion **206** of an implantable lead **104** (FIG. **4**). FIG. **7A** shows, among other things, a reinforcement design **1000** for increasing the axial strength between a distal electrode (e.g., **208***D*) and a lead body **202.** In this example, the lead body 202 includes at least a first **1004** and a second **1006** internal longitudinally extending lumen. A conductor **604** is received in, and extends along, the first lumen **1004** and electrically couples to the distal electrode **208***D* at a distal end thereof.

To provide additional axial strength to the distal electrode **208***D*/conductor **604** connection joint, the lead **104** (FIG. **4**) may include an axial support member **1002** received in the second lumen **1006.** The axial support member **1002** extends from an axial support member proximal end portion **1008** to an axial support member distal end portion **1010.** As shown, the axial support member proximal end portion **1008** is coupled with a first retaining member **1012,** while the axial support member distal end portion **1010** is coupled with a second retaining member **1014.** The retaining members **1012, 1014** are engaged with the lead body **202** and the distal electrode **208***D*, respectively, thereby providing the additional axial support to the distal electrode/conductor joint.

Many options are possible for the reinforcement design **1000.** In one example, the axial support member **1002** is a cable. In another example, the first **1012** and the second **1014** retaining members include at least one of a crimped tube, a swaged tube, a welded tube, or a brazed tube (i.e., a couplable tube). In one such example, the distal electrode **208***D* is placed over an outer surface of the crimped or swaged tube and laser welded thereto. In another example, an outer diameter of the first retaining member **1012** is greater than a diameter of the second lumen **1006** (see FIG. **7B**). To make room for such larger retaining member **1012,** the lumen **1006** may be cut or ablated in the placement vicinity of the member **1012.** In such an example, the distal electrode **208***D* is held in place because the first retaining member cannot slide through the second lumen **1006** due to its larger size. In addition, the lead body **202** may be fused around the retaining member **1012** to provide further retainment strength. In yet another example, a plug member **1016** (e.g., a polyurethane filament) or other sealant means is inserted in the second lumen **1006** proximal to the first retaining member **1012** and adhered to the lead body **202** (e.g., by heat fusing techniques) to electrically isolate the reinforcement member **1012** and the electrode **208***D* from a more proximal conductor in a second lumen. In further examples, an outer surface of distal electrode **208***D* is isodiametric or non-isodiametric with an outer surface of the lead body **202,** or one or more knots are used in conjunction with one or both of the first and the second retaining members.

FIG. **7B** is a transverse cross-sectional view of an implantable lead **104** (FIG. **4**) taken along line **10B-10B** of FIG. **7A**. FIG. **7B** shows, among other things, an outer diameter of the first retaining member **1012** greater than a diameter of the second lumen **1006** of the lead body **202** and covered by fusing of the same thereby precluding the retaining member **1012** from being pulled through the lumen **1006** without high force. As discussed above, the axial supporting member **1002** is coupled with the first retaining member **1012** on the axial supporting member proximal end **1008** and coupled with the second retaining member **1014** (FIG. **7A**) on the axial supporting member distal end **1010.** As further discussed above, the distal electrode **208***D* may be laser welded or otherwise secured to the second retaining member **1014** (FIG. **7A**). Accordingly, the axial load placed on distal electrode **208***D* is supported, at least in part, by the reinforcement design **1000.** As also shown in FIG. **7***B*, a weld ring **1050** coupled to the conductor **604** on an inner surface and fused to the lead body **202** on an outer surface may also be used to provide axial support to the distal electrode **208***D*/conductor **604** connection joint.

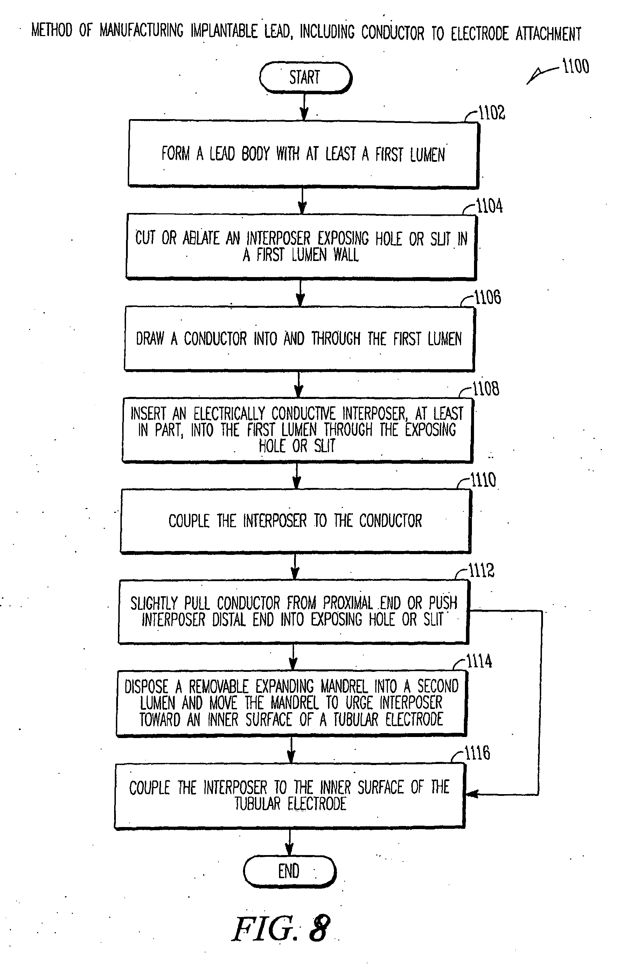

FIG. **8** illustrates a method **1100** of manufacturing an implantable lead. At **1102,** a lead body extending form a lead proximal end to a lead distal end is formed. Forming the lead body includes forming at least a first longitudinally extending lumen therein. In varying examples, wall portions of the first lumen are sized and shaped to urge an appropriately larger sized element (e.g., an electrically conductive interposer - see **1108**) placed in the first lumen outward toward a tubular electrode co-axial with, and overlying portions of, the lead body. At **1104,** an interposer exposing hole or slit is cut or ablated in a first lumen wall opposite the urging wall portions to receive the interposer and allow such element to electrically couple a conductor (see **1106**) and the tubular electrode. At **1106,** the conductor is drawn into and through the first lumen

At **1108,** the electrically conductive interposer is inserted into the first lumen such that the conductor is located therewithin (i.e., the (tubular) interposer is slid onto at least a portion of the conductor). At **1110,** the electrically conductive interposer is coupled to the conductor. This may initially include, among other things, removing an insulative coating on an outer side of the conductor. In one example, the electrically conductive interposer is coupled to the conductor via crimping, swaging, welding, or brazing. In one such example, the electrically conductive interposer is crimped to the conductor such that an outer surface of the electrically conductive interposer mates with a sized and shape of an inner surface of the tubular electrode, thereby providing a more conducive juncture for the process of **1112.**

At **1112,** the conductor is slightly pulled from a conductor proximal end or an interposer distal end is pushed into the exposing hole or slit so that the interposer is fully within the outer boundaries of the lead body. Optionally, at **1114,** a removable expanding mandrel may be disposed into a second lumen to urge the interposer outward toward the inner surface of the tubular electrode. At **1116,** the interposer is coupled to the inner surface of the tubular electrode via welding, brazing, conductive adhesives, or other suitable techniques.

The leads and methods described herein provide numerous advantages over conventional lead designs including secure electrical and mechanical connection between a conductor, such as a multi-strand conductor cable, and an electrode (e.g., a ring electrode or a multi-filar shock coil electrode). In addition, the leads and methods provide axial reinforcement between a distal electrode and a lead body. Furthermore, the leads and methods allow for the creation of a smaller-sized lead (e.g., sub-5 French), which may advantageously provide for easier and deeper lead delivery and lower sensing/stimulating thresholds. In one such example, the present leads and methods provide a relatively small-sized lead with multiple conductors and electrodes.

Several other advantages are also made possible by the present leads and methods. As one example, the leads and methods reduce or eliminate the reliance on adhesives to couple a conductor to an electrode. Advantageously, by reducing or eliminating reliance on adhesives, manufacturing efficiency can be increased (e.g., don't need to wait for adhesives to cure), and conductor/electrode joint failure caused by adhesive bond strength decreasing over time (e.g., due to reactions with bodily fluids or improper adhesive preparation) can be reduced or eliminated.

It is to be understood that the above description is intended to be illustrative, and not restrictive. It should be noted that the above text discusses, among other things, interconnections of implantable lead conductors and electrodes for use in cardiac situations; however, the present leads and methods are not so limited. Many other embodiments and contexts, such as for non-cardiac nerve and muscle situations or external situations, will be apparent to those of skill in the art upon reviewing the above description. The scope should, therefore, be determined with reference to the appended claims.

## Claims

1. A lead comprising:
- a lead body (202) extending from a lead proximal end portion to a lead distal end portion (204) and having a lead intermediate portion (302) therebetween, the lead body (202) including one or more longitudinally extending (606, 608, 610, 612) lumens;
- a cable conductor (602) received in, and extending along, a first lumen (610); and
- a tubular electrode (618) co-axial with, and overlying portions of, the lead body (202);
**characterised in that** the lead further comprises
- an electrically conductive interposer (614) having an inner surface coupled with the cable conductor (602) and an outer surface, the interposer (614) received, at least in part, in the first lumen (610); and
wherein a first portion of a first lumen wall is adapted to compressively urge the outer surface of the interposer (614), at least in part, through an interposer exposing aperture (650) in a second portion of the first lumen wall toward an inner surface portion of the tubular electrode (618) adjacent the cable conductor, such that a portion of the interposer is intermediately positioned between the cable conductor and the adjacent inner surface portion of the tubular electrode.

2. The lead of claim 1, wherein the interposer exposing aperture (650) in the second portion of the first lumen wall is disposed opposite the first portion of the first lumen wall urging the interposer.

3. The lead of claims 1 or 2, wherein the interposer (614) is coupled with the inner surface of the tubular electrode (618) adjacent the cable conductor.

4. The lead of any of claims 1-3, wherein an outer surface of the interposer (614) mates with a portion of the inner surface of the tubular electrode (618) adjacent the cable conductor.

5. The lead of any of claims 1-4, wherein the lead body (202) comprises an elastic polymer.

6. A method comprising:
forming a lead body (202) extending from a lead proximal end portion (204) to a lead distal end portion (206) and having a lead intermediate portion (302) therebetween;
forming one or more longitudinally extending lumens (606, 608, 610, 612) within the lead body (202), including forming a first lumen (610) having:
a first lumen wall portion sized and shaped to urge a tubular electrically conductive interposer (614) toward an inner surface of a tubular electrode (618) co-axial with, and overlying portions of, the lead body (202), and
a second lumen wall portion comprising an interposer exposing aperture (650) sized and shaped to allow electrical connection between the tubular interposer (614) and the tubular electrode (618);
drawing a conductor (602) into and through the first lumen (610);
inserting the tubular interposer (614), at least in part, into the first lumen (610), including placing the tubular interposer over the conductor and positioning a portion of the tubular interposer intermediate the conductor and an inner surface portion of the tubular electrode adjacent the conductor; and
coupling the tubular interposer (614) with the conductor (602).

7. The method of claim 6, further comprising compression fitting the tubular interposer (614) with the inner surface of the tubular electrode (618).

8. The method of claim 7, wherein compression fitting the tubular interposer (614) with the inner surface of the tubular electrode (618) includes disposing a removable expanding mandrel (652) in a second lumen (612) positioned adjacent the first lumen (610); and
urging the tubular interposer (614) toward the inner surface (616) of the electrode (618) via movement of the mandrel (652).

9. The method of any of claims 6-8, further comprising coupling an outer surface of the tubular interposer (614) with the inner surface (616) of the tubular electrode (618) adjacent the conductor.

10. The lead of any of claims 1-5, wherein the interposer exposing aperture (650) includes a hole.

11. The lead of any of claims 1-5, wherein the interposer exposing aperture (650) includes a slit.

12. The lead of any of claims 1-5, 10 or 11, wherein the interposer (614) is crimped, swaged, welded, or brazed to the conductor (602).

13. The lead of any of claims 1-5 or 10-12, wherein an outer surface of the tubular electrode (618) is substantially isodiametric with an outer surface of the lead body (202).

14. The lead of any of claims 1-5 or 10-13, wherein an outer diameter of the interposer (614) is greater than a diameter of the first lumen (610), but bounded by the first lumen (610).

15. The lead of any of claims 1-5 or 10-14, further comprising an axial support member (1002) received in a second lumen (1006), distinct from the first lumen (1004), the axial support member (1002) extending from an axial support member proximal end portion (1008) coupled with a first retaining member (1012) to an axial support member distal end portion (1010) coupled with a second retaining member (1014); and
wherein the first retaining member (1012) is engaged with the lead body (202) and the second retaining member (1014) is engaged with the tubular electrode (208D).

16. The lead of claim 15, wherein the tubular electrode (208D) is disposed at the lead distal end portion.

17. The lead of claims 15 or 16, wherein one or both of the first retaining member (1012) or the second retaining member (1014) includes a couplable tube.

18. The lead of any of claims 15-17, wherein an outer diameter of one or both of the first retaining member (1012) or the second retaining member (1014) is greater than a diameter of the second lumen (1006).

19. The lead of any of claims 1-5 or 10-18, wherein the interposer (614) comprises a tubular shape.

20. The lead of claim 3, wherein an outer surface of the interposer (614) is welded to the inner surface (616) of the tubular electrode (618).

21. The method of claim 9, wherein coupling the tubular interposer (614) to the inner surface (616) of the tubular electrode (618) includes welding or brazing the tubular interposer (614) to the tubular electrode (618).

22. The method of any of claims 6-8 or 21, wherein coupling the tubular interposer (614) to the conductor (602) includes crimping, swaging, welding, or brazing.

23. The method of any of claims 6-8, 21 or 22, further comprising inserting an axial support member (1002) in a second lumen (1006), distinct from the first lumen (1004), and engaging an axial support member proximal end portion (1008) with the lead body (202) and an axial support member distal end portion (1010) with a distal tubular electrode (208D).

## Patentansprüche

1. Leiter, welcher aufweist:
einen Leiterkörper (202), der sich von einem proximalen Endbereich des Leiters zu einem distalen Endbereich (204) des Leiters erstreckt und einen Leiterzwischenbereich (302) zwischen diesen hat, wobei der Leiterkörper (202) ein oder mehrere sich in Längsrichtung erstreckende Lumen (606, 608, 610, 612) enthält;
einen Kabelleiter (602), der in einem ersten Lumen (610) aufgenommen ist und sich entlang diesem erstreckt; und
eine rohrförmige Elektrode (618), die koaxial mit dem Leiterkörper (202) angeordnet ist und über Teilen von diesem liegt;
**dadurch gekennzeichnet, dass** der Leiter weiterhin aufweist:
ein elektrisch leitendes Zwischenstück (614) mit einer inneren Oberfläche, die mit dem Kabelleiter (602) gekoppelt ist, und einer äußeren Oberfläche, wobei das Zwischenstück (614) zumindest teilweise in dem ersten Lumen (610) aufgenommen ist; und
wobei ein erster Bereich einer ersten Lumenwand ausgebildet ist zum zusammenpressenden Drücken gegen die äußere Oberfläche des Zwischenstücks (614) zumindest teilweise durch eine das Zwischenstück freilegende Öffnung (650) in einem zweiten Bereich der ersten Lumenwand zu einem inneren Oberflächenbereich der rohrförmigen Elektrode (618) angrenzend an den Kabelleiter hin, derart, dass ein Bereich des Zwischenstücks zwischen dem Kabelleiter und dem angrenzenden inneren Oberflächenbereich der rohrförmigen Elektrode positioniert ist.

2. Leiter nach Anspruch 1, bei dem die das Zwischenstück freilegende Öffnung (650) in dem zweiten Bereich der ersten Lumenwand gegenüber dem gegen das Zwischenstück drückenden ersten Bereich der ersten Lumenwand angeordnet ist.

3. Leiter nach Anspruch 1 oder 2, bei dem das Zwischenstück (614) mit der inneren Oberfläche der rohrförmigen Elektrode (618) angrenzend an den Kabelleiter gekoppelt ist.

4. Leiter nach einem der Ansprüche 1 bis 3, bei dem eine äußere Oberfläche des Zwischenstücks (614) einem Bereich der inneren Oberfläche der rohrförmigen Elektrode (618) angrenzend an den Kabelleiter angepasst ist.

5. Leiter nach einem der Ansprüche 1 bis 4, bei dem der Leiterkörper (202) ein elastisches Polymer aufweist.

6. Verfahren, welches aufweist:
Bilden eines Leiterkörpers (202), der sich von einem proximalen Endbereich (204) des Leiters zu einem distalen Endbereich (206) des Leiters erstreckt und einen Leiterzwischenbereich (302) zwischen diesen h at;
Bilden eines oder mehrerer sich in Längsrichtung erstreckender Lumen (606, 608, 610, 612) innerhalb des Leiterkörpers (202), enthaltend das Bilden eines ersten Lumens (610) mit
einem ersten Lumenwandbereich, der bemessen und geformt ist, ein rohrförmiges, elektrisch leitendes Zwischenstück (614) zu einer inneren Oberfläche einer rohrförmigen Elektrode (618), die mit dem Leiterkörper (202) koaxial ist und über Bereichen von diesem liegt, hin zu drücken, und
einem zweiten Lumenwandbereich, aufweisend eine das Zwischenstück freilegende Öffnung (650), die so bemessen und geformt ist, dass sie eine elektrische Verbindung zwischen dem rohrförmigen Zwischenstück (614) und der rohrförmigen Elektrode (618) ermöglicht;
Ziehen eines Leiters (602) in und durch das erste Lumen (610);
Einsetzen des rohrförmigen Zwischenstücks (614) zumindest teilweise in das erste Lumen (610), enthaltend das Anordnen des rohrförmigen Zwischenstücks über dem Leiter und das Positionieren eines Teils des rohrförmigen Zwischenstücks zwischen dem Leiter und einem inneren Oberflächenbereich der rohrförmigen Elektrode angrenzend an den Leiter; und
Koppeln des rohrförmigen Zwischenstücks (614) mit dem Leiter (602).

7. Verfahren nach Anspruch 6, weiterhin aufweisend das Herstellen einer Druckpassung zwischen dem rohrförmigen Zwischenstück (614) und der inneren Oberfläche der rohrförmigen Elektrode (618).

8. Verfahren nach Anspruch 7, bei dem das Herstellen der Druckpassung zwischen dem rohrförmigen Zwischenstück (614) und der inneren Oberfläche der rohrförmigen Elektrode (618) das Anordnen einer entfernbaren, sich ausdehnenden Spindel (652) in einem zweiten Lumen (612), das angrenzend an das erste Lumen (610) positioniert ist;
und
das Drücken des rohrförmigen Zwischenstücks (614) zu der inneren Oberfläche (616) der Elektrode (618) hin über eine Bewegung der Spindel (652) enthält.

9. Verfahren nach einem der Ansprüche 6 - 8, weiterhin aufweisend das Koppeln einer äußeren Oberfläche des rohrförmigen Zwischenstücks (614) mit der inneren Oberfläche (616) der rohrförmigen Elektrode (618) angrenzend an den Leiter.

10. Verfahren nach einem der Ansprüche 1-5, bei dem die das Zwischenstück freilegende Öffnung (650) ein Loch enthält.

11. Verfahren nach einem der Ansprüche 1-5, bei dem die das Zwischenstück freilegende Öffnung (650) einen Schlitz enthält.

12. Leiter nach einem der Ansprüche 1-5, 10 oder 11, bei dem das Zwischenstück (614) auf den Kabelleiter (602) gefalzt, geschmiedet, geschweißt oder hartgelötet ist.

13. Leiter nach einem der Ansprüche 1-5 oder 10 - 12, bei dem eine äußere Oberfläche der rohrförmigen Elektrode (618) im Wesentlichen isodiametral mit einer äußeren Oberfläche des Leiterkörpers (202) ist.

14. Leiter nach einem der Ansprüche 1 - 5 oder 10 - 13, bei dem ein äußerer Durchmesser des Zwischenstücks (614) größer als ein Durchmesser des ersten Lumens (610), aber durch das erste Lumen (610) begrenzt ist.

15. Leiter nach einem der Ansprüche 1 - 5 oder 10 - 14, weiterhin aufweisend ein axiales Stützteil (1002), das in einem zweiten Lumen (1006) getrennt von dem ersten Lumen (1004) aufgenommen ist, wobei das axiale Stützteil (1002) sich von einem proximalen Endbereich (1008) des axialen Stützteils, der mit einem ersten Halteteil (1012) gekoppelt ist, bis zu einem distalen Endbereich (1010) des axialen Stützteils, der mit einem zweiten Halteteil (1014) gekoppelt ist, erstreckt; und wobei das erste Halteteil (1012) in Eingriff mit dem Leiterkörper (202) ist und das zweite Halteteil (1014) in Eingriff mit der rohrförmigen Elektrode (208D) ist.

16. Leiter nach Anspruch 15, bei dem die rohrförmige Elektrode (208D) sich an dem distalen Endbereich des Leiters befindet.

17. Leiter nach den Ansprüche 15 oder 16, bei dem eines oder beide von dem ersten Halteteil (1012) oder dem zweiten Halteteil (1014) ein koppelbares Rohr enthält.

18. Leiter nach einem der Ansprüchen 15 - 17, bei dem ein äußerer Durchmesser von einem oder beiden von dem ersten Halteteil (1012) oder dem zweiten Halteteil (1014) größer als ein Durchmesser des zweiten Lumens (1006) ist.

19. Leiter nach einem der Ansprüche 1 - 5 oder 10 - 18, bei dem das Zwischenstück (614) eine Rohrform aufweist.

20. Leiter nach Anspruch 3, bei dem eine äußere Oberfläche des Zwischenstücks (614) an die innere Oberfläche (616) der rohrförmigen Elektrode (618) geschweißt ist.

21. Verfahren nach Anspruch 9, bei dem das Koppeln des rohrförmigen Zwischenstücks (614) an die innere Oberfläche (616) der rohrförmigen Elektrode (618) das Schweißen oder Hartlöten des rohrförmigen Zwischenstücks (614) an die rohrförmige Elektrode (618) enthält.

22. Verfahren nach einem der Ansprüche 6-8 oder 21, bei dem das Koppeln des rohrförmigen Zwischenstücks (614) an den Kabelleiter (602) das Falzen, Schmieden, Schweißen oder Hartlöten enthält.

23. Verfahren nach einem der Ansprüche 6 - 8, 21 oder 22, weiterhin aufweisend das Einsetzen eines axialen Stützteils (1002) in ein zweites Lumen (1006) getrennt von dem ersten Lumen (1004) und das Ineingriffbringen eines proximalen Endbereichs (1008) des axialen Stützteils mit dem Leiterkörper (202) sowie eines distalen Endbereichs (1010) des axialen Stützteils mit einer distalen rohrförmigen Elektrode (208D) aufweist.

## Revendications

1. Connexion comprenant :
un corps de connexion (202) qui s'étend depuis une partie d'extrémité proximale de connexion jusqu'à une partie d'extrémité distale de connexion (204) et qui comporte une partie intermédiaire de connexion (302) entre, le corps de connexion (202) incluant une ou plusieurs lumière(s) s'étendant longitudinalement (606, 608, 610, 612) ;
un câble conducteur (602) reçu dans une première lumière (610) et s'étendant le long de celle-ci ; et
une électrode tubulaire (618) coaxiale au corps de connexion (202) et s'étendant sur des parties de celui-ci,
**caractérisée en ce que** la connexion comprend en outre :
un interposeur électriquement conducteur (614) comportant une surface interne couplée au câble conducteur (602) et une surface externe, l'interposeur (614) étant reçu, au moins en partie, dans la première lumière (610) ; et dans laquelle
une première partie d'une paroi de première lumière est adaptée de manière à pousser par compression la surface externe de l'interposeur (614), au moins en partie, au travers d'une ouverture d'exposition d'interposeur (650) ménagée dans une seconde partie de la paroi de première lumière en direction d'une partie de surface interne de l'électrode tubulaire (618) adjacente au câble conducteur de telle sorte qu'une partie de l'interposeur soit positionnée de façon intermédiaire entre le câble conducteur et la partie de surface interne adjacente de l'électrode tubulaire.

2. Connexion selon la revendication 1, dans laquelle l'ouverture d'exposition d'interposeur (650) ménagée dans la seconde partie de la paroi de première lumière est disposée à l'opposé de la première partie de la paroi de première lumière qui pousse l'interposeur.

3. Connexion selon la revendication 1 ou 2, dans laquelle l'interposeur (614) est couplé à la surface interne de l'électrode tubulaire (618) adjacente au câble conducteur.

4. Connexion selon l'une quelconque des revendications 1 à 3, dans laquelle une surface externe de l'interposeur (614) se conjugue avec une partie de la surface interne de l'électrode tubulaire (618) adjacente au câble conducteur.

5. Connexion selon l'une quelconque des revendications 1 à 4, dans laquelle le corps de connexion (202) comprend un polymère élastique.

6. Procédé comprenant :
la formation d'un corps de connexion (202) qui s'étend depuis une partie d'extrémité proximale de connexion (204) jusqu'à une partie d'extrémité distale de connexion (206) et qui comporte une partie intermédiaire de connexion (302) entre ;
la formation d'une ou de plusieurs lumière(s) s'étendant longitudinalement (606, 608, 610, 612) à l'intérieur du corps de connexion (202), incluant la formation d'une première lumière (610) comportant :
une première partie de paroi de lumière dimensionnée et conformée de manière à pousser un interposeur tubulaire électriquement conducteur (614) en direction d'une surface interne d'une électrode tubulaire (618) coaxiale au corps de connexion (202) et s'étendant sur des parties de celui-ci ;et
une seconde partie de paroi de lumière comprenant une ouverture d'exposition d'interposeur (650) dimensionnée et conformée de manière à permettre une connexion électrique entre l'interposeur tubulaire (614) et l'électrode tubulaire (618) ;
le fait de tirer un conducteur (602) à l'intérieur de la première lumière (610) et au travers de celle-ci ;
l'insertion de l'interposeur tubulaire (614), au moins en partie, à l'intérieur de la première lumière (610), incluant le placement de l'interposeur tubulaire au dessus du conducteur et le positionnement d'une partie de l'interposeur tubulaire de telle sorte qu'elle soit intermédiaire entre le conducteur et une partie de surface interne de l'électrode tubulaire adjacente au conducteur ; et
le couplage de l'interposeur tubulaire (614) au conducteur (602).

7. Procédé selon la revendication 6, comprenant en outre l'ajustage par compression de l'interposeur tubulaire (614) avec la surface interne de l'électrode tubulaire (618).

8. Procédé selon la revendication 7, dans lequel l'ajustage par compression de l'interposeur tubulaire (614) avec la surface interne de l'électrode tubulaire (618) inclut :
la disposition d'un mandrin d'extension amovible (652) dans une seconde lumière (612) positionnée de manière à être adjacente à la première lumière (610) ;et
la poussée de l'interposeur tubulaire (614) en direction de la surface interne (616) de l'électrode (618) via un déplacement du mandrin (652).

9. Procédé selon l'une quelconque des revendications 6 à 8, comprenant en outre le couplage d'une surface externe de l'interposeur tubulaire (614) à la surface interne (616) de l'électrode tubulaire (618) adjacente au conducteur.

10. Connexion selon l'une quelconque des revendications 1 à 5, dans laquelle l'ouverture d'exposition d'interposeur (650) inclut un trou.

11. Connexion selon l'une quelconque des revendications 1 à 5, dans laquelle l'ouverture d'exposition d'interposeur (650) inclut une fente.

12. Connexion selon l'une quelconque des revendications 1 à 5, 10 ou 11, dans laquelle l'interposeur (614) est forgé, estampé, soudé ou brasé sur le conducteur (602).

13. Connexion selon l'une quelconque des revendications 1 à 5 ou 10 à 12, dans laquelle une surface externe de l'électrode tubulaire (618) est sensiblement isodiamétrique avec une surface externe du corps de connexion (202).

14. Connexion selon l'une quelconque des revendications 1 à 5 ou 10 à 13, dans laquelle un diamètre externe de l'interposeur (614) est supérieur à un diamètre de la première lumière (610) mais est limité par la première lumière (610).

15. Connexion selon l'une quelconque des revendications 1 à 5 ou 10 à 14, comprenant en outre :
un élément de support axial (1002) reçu dans une seconde lumière (1006) distincte de la première lumière (1004), l'élément de support axial (1002) s'étendant depuis une partie d'extrémité proximale d'élément de support axial (1008) couplée à un premier élément de retenue (1012) jusqu'à une partie d'extrémité distale d'élément de support axial (1010) couplée à un second élément de retenue (1014) ; et dans laquelle
le premier élément de retenue (1012) est engagé avec le corps de connexion (202) et le second élément de retenue (1014) est engagé avec l'électrode tubulaire (208D).

16. Connexion selon la revendication 15, dans laquelle l'électrode tubulaire (208D) est disposée au niveau de la partie d'extrémité distale de connexion.

17. Connexion selon la revendication 15 ou 16, dans laquelle l'un des éléments que sont le premier élément de retenue (1012) et le second élément de retenue (1014) ou les deux inclu(en)t un tube de couplage.

18. Connexion selon l'une quelconque des revendications 15 à 17, dans laquelle un diamètre externe de l'un des éléments que sont le premier élément de retenue (1012) et le second élément de retenue (1014) ou des deux est plus grand qu'un diamètre de la seconde lumière (1006).

19. Connexion selon l'une quelconque des revendications 1 à 5 ou 10 à 18, dans laquelle l'interposeur (614) présente une forme tubulaire.

20. Connexion selon la revendication 3, dans laquelle une surface externe de l'interposeur (614) est soudée sur la surface interne (616) de l'électrode tubulaire (618).

21. Procédé selon la revendication 9, dans lequel le couplage de l'interposeur tubulaire (614) à la surface interne (616) de l'électrode tubulaire (618) inclut le soudage ou le brasage de l'interposeur tubulaire (614) sur l'électrode tubulaire (618).

22. Procédé selon l'une quelconque des revendications 6 à 8 ou 21, dans lequel le couplage de l'interposeur tubulaire (614) au conducteur (602) inclut le forgeage, l'estampage, le soudage ou le brasage.

23. Procédé selon l'une quelconque des revendications 6 à 8, 21 ou 22, comprenant en outre l'insertion d'un élément de support axial (1002) dans une seconde lumière (1006) distincte de la première lumière (1004) et l'engagement d'une partie d'extrémité proximale d'élément de support axial (1008) avec le corps de connexion (202) et d'une partie d'extrémité distale d'élément de support axial (1010) avec une électrode tubulaire distale (208D).
